# EUROPÄISCHE PATENTSCHRIFT

(11) **EP 0 752 010 B1**
(45) Veröffentlichungstag und Bekanntmachung des Hinweises auf die Patenterteilung: **14.10.1998**
(21) Anmeldenummer: 95913109.5
(22) Anmeldetag: 14.03.1995
(51) Int. Cl.: C14C 9/00

(54) **VERWENDUNG VON ALKYLENDIAMINTETRAPROPIONSÄUREN ZUR FETTENDEN AUSRÜSTUNG VON LEDER**
USE OF ALKYLENE DIAMINE TETRAPROPIONIC ACIDS FOR THE OILING OFF OF LEATHERS
UTILISATION D'ACIDES ALKYLENE-DIAMINOTETRAPROPIONIQUES POUR METTRE DES CUIRS EN HUILE

(30) Priorität: 23.03.1994 DE 4409926
(43) Veröffentlichungstag der Anmeldung: 08.01.1997
(73) Patentinhaber: Henkel Kommanditgesellschaft auf Aktien, 40191 Düsseldorf (DE)
(72) Erfinder: ZAUNS-HUBER, Rudolf, D-40589 Düsseldorf (DE); WOLTER, Fredi, D-41189 Mönchengladbach (DE); UPHUES, Günter, D-40789 Monheim (DE); PRINZ, Wolfgang, D-40789 Monheim (DE); SCHENKER, Gilbert, D-40699 Erkrath (DE)
(86) Internationale Anmeldenummer: EP9500946
(87) Internationale Veröffentlichungsnummer: WO9525816

(56) Entgegenhaltungen:
- EP-A- 0 184 741
- GB-A- 1 048 321
- DATABASE WPI Week 7317, Derwent Publications Ltd., London, GB; AN 73-24035U & JP,A,48 012 473 (KANEGAFUCHI SPINNING)

## Beschreibung

### Gebiet der Erfindung

Die Erfindung betrifft die Verwendung der untenstehend näher bezeichneten Alkylendiamintetrapropionsäuren zur fettenden Ausrüstung von Leder. Dabei zeichnen sich diese Verbindungen insbesondere dadurch aus, daß sie den mit ihnen behandelten Ledern bzw. Pelzen gute Wasch- und Reinigungs-Echtheit, Fogging-Echtheit und gute Wasserfestigkeit verleihen.

### Stand der Technik

Die Fettung pflanzlich und/oder mineralisch gegerbter Leder beziehungsweise Pelzfelle ist ein essentieller Verfahrensschritt in der Ausrüstung zum gebrauchsfertigen Wertstoff. Die Form der Fettverteilung in der Hautsubstanz und das Ausmaß der Einbindung der Fettkomponenten in die Hautsubstanz beeinflussen die Eigenschaften und die Gebrauchsfähigkeit der Fertigprodukte entscheidend. Es besteht dabei umfangreiches Fachwissen zu möglichen Interaktionen zwischen den Fettkomponenten einerseits und der gegerbten, sowie Restgerbstoffe enthaltenden Hautsubstanz andererseits. Der spezielle Aufbau der Fettungsmittel - beispielsweise das Ausmaß ihrer lipophilen Gruppen und gegebenenfalls vorliegenden Reaktivgruppen zur Umsetzung mit geeigneten Reaktivbestandteilen im gegerbten Leder - bestimmen unter anderem die Dauerhaftigkeit und Wirkung der fettenden Ausrüstung im praktischen Gebrauch der Leder- und Pelzwaren.

Ein für die Praxis sehr wichtiges Bedürfnis besteht darin, fettende Substanzen bzw. Ausrüstungsmittel zur Verfügung zu stellen, die in der gegerbten Hautsubstanz so zuverlässig gebunden werden können, daß eine für die praktischen Bedürfnisse hinreichende Wasch- und Reinigungsbeständigkeit der Leder- und Pelzwaren sichergestellt ist. Hochwertige Lederwaren, beispielsweise aus der Bekleidungsindustrie, sollen dabei sowohl der wäßrig-tensidischen Wäsche als auch gegebenenfalls einer chemischen Reinigung ohne wesentliche Qualitätseinbuße zugänglich sein.

Für Leder, die im Innenbereich von Autos und Flugzeugen Verwendung finden, besteht darüber hinaus Bedarf, über Substanzen zur fettenden Ausrüstung zu verfügen, die Fogging-echt sind. Unter "Fogging" ist zu verstehen, daß im Laufe der Zeit flüchtige Substanzen aus dem Leder entweichen und sich in unerwünschter Weise niederschlagen, z.B. auf Windschutzscheiben. Unter Fogging-echten Substanzen ist zu verstehen, daß diese Substanzen zum einen selbst so fest im Innern des Leders gebunden sind, daß sie praktisch nicht flüchtig sind, zum anderen, daß diese Substanzen die Fogging-Charakteristik üblicher Fettungsmittel bzw. Fettungsmittelbestandteile verbessern, d.h. deren Fogging-Werte reduzieren.

Schließlich wird für Sonderfälle die weiterführende Bedingung hinreichender Wasserdichtigkeit des fertig ausgerüsteten Leders gewünscht. Zur wasserdichten Ausrüstung von Leder oder Pelzen sind insbesondere drei Verfahrensprinzipien bekannt: (1) Imprägnierung durch Einlagerung wasserunlöslicher Verbindungen, zum Beispiel feste Fette, Wachse oder spezielle Polymere; (2) Imprägnierung durch Einlagerung wasserquellender Verbindungen, die bei Wasseraufnahme hochviskose Emulsionen bilden und die Faserzwischenräume des Leders verstopfen, zum Beispiel spezielle Emulgatoren vom W/O-Typ und (3) Behandlung mit hydrophobierend wirkenden Verbindungen, zum Beispiel Aluminium-, Chrom- und/oder Zirkonkomplexe, Silkone oder organische Fluorverbindungen.

Die DE 1 669 347 beschreibt die Verwendung von wasseremulgierbaren Sulfobernsteinsäure-Halbestern zum Fetten von Leder, wobei jedoch noch keine Wasserdichteffekte erzielt werden.

Gegenstand der EP 193 832 ist ein Verfahren zur Herstellung wasserdichter Leder oder Pelze unter Verwendung von Sulfobernsteinsäure-Monoestern in Kombination mit imprägnierenden und/oder hydrophobierenden Fettungsmitteln.

Die DE 37 17 961 beschreibt ein Verfahren zur Herstellung von N,N-di-substituierten β-Aminopropionsäuren und ihre Verwendung unter anderem zur Hydrophobierung von Leder und Pelzen. Die Herstellung dieser Verbindungen geschieht dabei in zwei Schritten derart, daß zunächst primäre Alkylamine an Acryl- bzw. Methacrylsäure angelagert werden und die dabei entstandenen N-Alkylaminopropionsäuren mit Carbonsäureanhydriden, Carbonsäurechloriden, Sulfonsäurechlorid, Isocyanaten, Halogencarbonsäuren oder Acryl- bzw. Methacylsäuren umgesetzt werden. Die Produkte können gewünschtenfalls anschließend noch - wenigstens teilweise - neutralisiert werden.

In der neueren Patentliteratur werden für die fettende Ausrüstung von insbesondere mineralisch gegerbten Ledern und Pelzen amphiphile Mittel beschrieben, die bestimmt ausgewählte Co-Oligomere von einerseits hydrophoben beziehungsweise oleophilen Monomeren und andererseits hydrophilen Monomerbestandteilen darstellen. Amphiphile Mittel dieser Art können in Form wäßriger Dispersionen, Emulsionen und/oder Lösungen nach Abschluß der Hauptgerbung in die auszurüstenden Leder oder Felle eingearbeitet - beispielsweise eingewalkt - werden. Insbesondere im Fall mineralisch gegerbter Leder oder Pelzfelle können diese amphiphilen Mittel gleichzeitig die Funktion der Nachgerbung übernehmen. Es kann schließlich eine abschließende Fixierung der amphiphilen Mittel mit insbesondere Mineralgerbstoffen vorgesehen sein. Die jüngere Patentliteratur beschreibt Hilfsstoffe der hier betroffenen Art:

So sind zum Beispiel in der EP 372 746 entsprechende Mittel und ihre Anwendung beschrieben, wobei die amphiphilen Copolymere aus einem überwiegenden Anteil wenigstens eines hydrophoben Monomeren und einem untergeordneten Anteil wenigstens eines copolymerisierbaren hydrophilen Monomeren gebildet sind. Als hydrophobe Monomere sind aufgezählt: langkettige Alkyl(meth)acrylate, langkettige Alkoxy- oder Alkylphenoxy(polyethylen-oxid)-(meth)acrylate, pimäre Alkene, Vinylester von lankettigen Alkylcarbonsäuren und deren Gemische. Die in geringerem Anteil vorliegenden hydrophilen Comonomeren sind ethylenisch ungesättigte wasserlösliche Säuren oder hydrophile basische Comonomere. Das Molekulargewicht (Gewichtsmittel) der Copolymeren liegt im Bereich von 2.000 bis 100.000.

Die EP 412 389 beschreibt als Mittel zum Hydrophobieren von Leder und Pelzfellen den Einsatz von Copolymerisaten, die durch radikalische Copolymerisation von (a) C₈₋₄₀-Monoolefinen mit (b) ethylenisch ungesättigten C₄₋₈-Dicarbonsäureanhydriden nach Art einer Substanzpolymerisation bei Temperaturen von 80 bis 300°C zu Copolymerisaten mit Molmassen von 500 bis 20.000 g/Mol, anschließende Solvolyse der Anhydridgruppen der Copolymerisate und zumindest partielle Neutralisation der bei der Solvolyse entstehenden Carboxylgruppen in wäßrigem Medium mit Basen hergestellt worden sind und die in Form von wäßrigen Dispersionen oder Lösungen vorliegen.

In der EP 418 661 wird zum gleichen Zweck die Verwendung von Copolymerisaten beschrieben, die (a) 50 bis 90 Gew.-% C₈₋₄₀-Alkyl(meth)acrylate, Vinylester von C₈₋₄₀-Carbonsäuren oder deren Mischungen und (b) 10 bis 50 Gew.-% monoethylenisch ungesättigte C₃₋₁₂-Carbonsäuren, monoethylenisch ungesättigte Dicarbonsäureanhydride, Halbester oder Halbamide von monoethylenisch ungesättigten C₄₋₁₂-Dicarbonsäuren, Amide von C₃₋₁₂-Monocarbonsäuren oder Mischungen davon einpolymerisiert enthalten und Molmassen von 500 bis 30.000 g/Mol besitzen.

Gegenstand der EP-A-498 632 ist schließlich ein Verfahren zur Herstellung von Leder mit verbesserter Fogging-Charakteristik. Dies wird erreicht durch Behandlung des Leders mit wäßrigen Dispersionen, die frei sind von organischen Lösungsmitteln und die ein amphiphiles Copolymer enthalten, das aus wenigstens einem hydrophilen Monomer und wenigstens einem hydrophoben Monomer besteht.

Schließlich sei darauf hingewiesen, daß es in der Ledertechnik weithin üblich ist, im Anschluß an den Hydrophobierungsschritt eine nachträgliche Fixierung des hydrophobierenden Wirkstoffs durchzuführen. Darunter versteht man einen Verfahrensschritt, durch den sichergestellt werden soll, daß die in das Leder eingetragene hydrophobierende Komponente an die Lederfasern gebunden wird. Üblicherweise wird eine solche Fixierung mit Chrom- und/oder Aluminiumsalzen durchgeführt. Ein Beispiel für diese Technik bietet die DE-A-35 07 241 bzw. die DE-A-36 20 780. In vielen Ländern verschärfen sich jedoch die Auflagen bezüglich des Chromgehalts im Abwasser. Bekanntlich wird aber bei Verwendung konventioneller Gerbstoffe ein nicht unerheblicher Teil von der Lederfaser nicht gebunden und gelangt deshalb über Wasch- und Spülvorgänge ins Abwasser. Beim Einsatz hochauszehrender Chromgerbstoffe ist dieser Anteil zwar geringer, für die Praxis in der Regel aber immer noch auf einem Niveau, das das Bedürfnis nach niedrigeren Werten entstehen läßt.

### Beschreibung der Erfindung

Aufgabe der vorliegenden Erfindung war es, Substanzen für die fettende Ausrüstung von Leder zur Verfügung zu stellen. Dabei sollten diese Substanzen insbesondere in der Lage sein, den mit ihnen behandelten Ledern bzw. Pelzen gute Waschechtheit, Reinigungs-Echtheit und Fogging-Echtheit zu verleihen.

Unter Waschechtheit und Reinigungs-Echtheit ist - wie oben bereits angedeutet und dem Fachmann geläufig - zu verstehen, daß die mit den entsprechenden Substanzen behandelten Leder bzw. Pelze durch die entsprechenden Reinigungsoperationen, z.B. durch das Waschen mit tensidischen Flotten in Waschmaschinen oder die chemische Reinigung in z.B. Perchlorethylen-haltigen Bädern, hinsichtlich ihrer Gebrauchseigenschaften nicht oder nicht wesentlich beeinträchtigt werden, also z.B. nicht schrumpfen.

Eine weitere Aufgabe bestand darin, Substanzen und Verfahrensweisen bereitzustellen, die aus wäßriger Flotte appliziert hydrophobierte Leder ergeben, ohne daß dabei eine nachträgliche Behandlung mit Mineralsalzen durchgeführt werden muß, um die genannte Abwasserkontamination zu vermeiden. Mit anderen Worten bestand Bedarf nach "selbst-fixierenden" Lederhydrophobierungsmitteln.

Die Aufgabe wurde erfindungsgemäß gelöst durch spezielle Alkylendiamintetrapropionsäuren der unten näher bezeichneten Struktur.

Gegenstand der vorliegenden Erfindung ist die Verwendung von **Alkylendiamintetrapropionsäuren der allgemeinen Formel (I)** 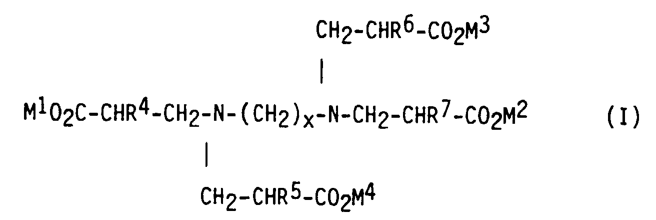 worin
- die Reste R⁴ bis R⁷ unabhängig voneinander Wasserstoff oder eine Methylgruppe und
- die Reste M¹ bis M⁴ unabhängig voneinander Wasserstoff, Alkalimetall, Erdalkalimetall, Ammonium, Alkylammonium oder Alkanolammonium und
- x eine Zahl von 12 bis 36 bedeuten und
- wobei die Alkylengruppe -(CH₂)ₓ- gegebenenfalls durch ein oder mehrere Alkyl-, Aryl- oder Alkarylgruppen substituiert sein kann, mit der Maßgabe, daß die Gesamtzahl der C-Atome dieser Substituenten 1 bis 40 beträgt,
zur fettenden Ausrüstung von Leder.

In einer bevorzugten Ausführungsform der vorliegenden Erfindung bedeuten die Reste M¹ bis M⁴ in der Formel für die Alkylendiamintetrapropionsäuren (I) - unabhängig voneinander - Wasserstoff oder ein Alkalimetall.

In bezug auf die Alkylengruppe, also die Gruppe -(CH₂)ₓ-, gilt, daß x vorzugsweise eine Zahl im Bereich von 12 bis 24 C-Atomen ist.

In der vorliegenden Erfindung kann die Alkylengruppe auch substituiert sein. Als Substituenten sind dabei insbesondere Alkylgruppen bevorzugt, wobei diese Alkylgruppen gesättigt oder olefinisch ungesättigt, geradkettig oder verzweigt sein können.

In einer weiteren Ausführungsform der Erfindung werden die Alkylendiamintetrapropionsäuren (I) in Kombination mit mindestens einem der nachstehend beschriebenen Aminopropionsäurederivate (II) und/oder Co-Oligomeren (III) eingesetzt.

Die **Aminopropionsäurederivate (II)** sind charakterisiert durch folgende Formel:

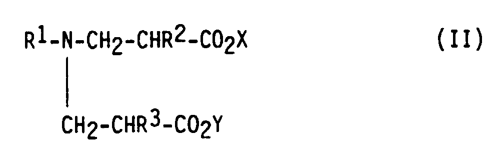

In der allgemeinen Formel (II) bedeuten
- der Rest R¹ eine gesättigte, geradkettige oder verzweigte Alkylgruppe mit 8 bis 22 C-Atomen,
- die Reste R² und R³ unabhängig voneinander Wasserstoff oder eine Methylgruppe und
- die Reste X und Y unabhängig voneinander Wasserstoff, Alkalimetall, Erdalkalimetall, Ammonium, Alkylammonium oder Alkanolammonium.

Dabei sind diejenigen Verbindungen (II) bevorzugt, in denen der Rest R¹ einen geradkettigen Alkylrest mit überwiegend 12 bis 18 C-Atomen, insbesondere mit 12 bis 14 C-Atomen, bedeutet. Die Reste R² und R³ bedeuten vorzugsweise Wasserstoff. In bezug auf die Reste X und Y gilt, daß diejenigen Verbindungen (I) bevorzugt sind, bei denen einer der Reste X und Y ein Alkalimetall und der andere Wasserstoff bedeutet.

Die **Co-Oligomeren (III)** sind dadurch charakterisiert, daß sie wasserdispergierbares und/oder wasseremulgierbar sind und aufgebaut sind aus
a) Fettcrotonaten und
b) radikalisch copolymerisierbaren hydrophilen ethylenisch ungesättigen Säuren und/oder deren Anhydriden, die auch
c) untergeordnete Mengen weiterer copolymerisierbarer Comonomere enthalten können.

Unter Fettcrotonaten sind dabei Ester der Crotonsäure (trans-2-Butensäure) mit C₁₀₋₄₀-Fettalkoholen zu verstehen.

Für die Co-Oligomeren (III) gilt, daß diejenigen Fettcrotonat-Bausteine (a) bevorzugt sind, die als Alkohol-Komponente C₁₂₋₂₄-Fettalkohole enthalten. Geradkettige Fettalkohole bzw. deren Mischungen sind dabei bevorzugt. Es hat sich allerdings gezeigt, daß auch die Mitverwendung von verzweigtkettigen Fettalkoholen im Fettcrotonat-Baustein (a) im Einzelfall zu interessanten Ergebnissen führen kann. So kann beispielsweise durch Mitverwendung von solchen verzweigtkettigen Alkoholen in beschränktem Ausmaß die Penetrationsfähigkeit des Co-Oligomeren (III) in die Faserstruktur des auszurüstenden Hautmaterials gefördert werden. Dabei ist es auch möglich, entsprechende verzweigte Alkohole einer kürzeren C-Zahl einzusetzen, wobei entsprechende Alkohole mit wenigstens 6 C-Atomen, vorzugsweise mit wenigstens 8 C-Atomen, in Betracht kommen. Ein wichtiger verzweigtkettiger Alkohol, der im Zusammenhang mit den Fettcrotonat-Bausteinen (a) zum Einsatz kommen kann, ist das 2-Ethylhexanol. Die Menge der mitzuverwendenden verzweigtkettigen insbesondere kürzeren Alkohole wird allerdings immer vergleichsweise gering sein. So werden in der Regel nicht mehr als 50 Gew.-%, vorzugsweise nicht mehr als 30 oder nicht mehr als 20 Gew.-% der in (a) vorliegenden Alkoholreste durch solche verzweigtkettigen insbesondere niederen Alkohole gebildet. Für das praktische Arbeiten haben sich - bezogen auf das oleophile Fettcrotonat (a) - Mengenverhältnisse von wenigstens etwa 90-95 Gew.-% der ausgeprägt oleophilen langkettigen Fettalkohole, insbesondere des Bereichs C₁₂₋₁₈, als besonders geeignete Komponenten zur Ausbildung der Crotonatester erwiesen.

Die in den amphiphilen Co-Oligomeren (III) zum Einsatz kommenden hydrophilen Komponenten (b) können den entsprechenden Bausteinen aus den Co-Oligomeren der zitierten EP 372 746, EP 412 389 und EP 418 661 entsprechen. Insbesondere die zuletzt genannte Druckschrift bringt hier ausführliche Angaben zu geeigneten Stoffklassen und zahlreichen speziellen Vertretern.

Besonders wichtige Bausteine (b) für die Co-Oligomeren (III) sind im Sinne der vorliegenden Erfindung ethylenisch ungesättigte Monocarbonsäuren und/oder ethylenisch ungesättigte Dicarbonsäuren und/oder deren Anhydride mit bevorzugt jeweils bis zu 12 C-Atomen. Die Dicarbonsäuren können auch wenigstens anteilsweise in Form ihrer Partialderivate mit einer Carboxylgruppe und einer derivatisierten Carbonsäuregruppe zum Beispiel als Dicarbonsäure-Halbester vorliegen.

Besonders interessante Vertreter aus der Klasse monoethylenisch ungesättigter C₃₋₁₂-Monocarbonsäuren sind beispielsweise Acrylsäure, Methacrylsäure und die Crotonsäure. Besonders geeignete Bausteine (b) können aber auch Dicarbonsäuren beziehungsweise deren Derivate, insbesondere deren Anhydride sein. Typische Vertreter sind die Maleinsäure, Fumarsäure, Itaconsäure, Glutaconsäure und entsprechende Anhydride.

Besondere Bedeutung kommt dabei dem Maleinsäureanhydrid zu. Fettcrotonate (a) und Maleinsäureanhydrid lassen sich in befriedigender Weise mit oder ohne Verwendung von Hilfslösungsmitteln zu Co-Oligomeren (III) eines leicht einstellbar niedrigen Molgewichtes umsetzen. Es ist dabei insbesondere möglich, etwa gleiche Molmengen an Maleinsäureanhydrid und Fettcrotonat miteinander zur Umsetzung zu bringen, so daß nach Solvolyse, insbesondere Hydrolyse der Maleinsäureanhydrid-Bausteine einerseits hinreichend hohe Konzentrationen an Carboxylgruppen für die Fixierung der Oligomeren in der Haut- beziehungsweise Faserstruktur zur Verfügung stehen, andererseits aber auch zuverlässig hohe Gehalte des oleophilen und damit fettenden und wasserabweisenden Fettcrotonat-Baustein eingebunden sind.

Neben oder anstelle dieser hydrophilen Bausteine (b) auf Basis von Carbonsäuren sind aber auch andere copolymerisierbare hydrophile Verbindungen verwendbar. In Betracht kommen hier insbesondere Sulfosäuregruppen enthaltende ethylenisch ungesättigte Monomere. Bekannte Vertreter dieser Art sind insbesondere entsprechende aliphatische und/oder aromatische Sulfonsäuren, wie Styrolsulfonsäure aber auch Verbindungen wie Vinylsulfonsäure, Allylsulfonsäure, Methallylsulfonsäure und dergleichen. Wie bereits angegeben kann der Baustein (b) des Co-Oligomeren (III) ganz oder teilweise aus solchen der Copolymerisation beziehungsweise Co-Oligomerisation zugänglichen Sulfosäureverbindungen gebildet sein.

Besonders bevorzugt sind solche Co-Oligomere (III) in denen die Bausteine (a) und (b) in Verhältnissen von 30 bis 90 Gew% (a) auf 70 bis 10 Gew.-% (b) vorliegen - Gew.-% hier bezogen auf die Summe von (a) und (b). Bevorzugte Bereiche für die Mischungsverhältnisse sind etwa 35 bis 80 Gew.-% (a) auf 65 bis 20 Gew.-% (b).

Neben den Bausteinen (a) und (b) können die amphiphilen Co-Oligomeren (III) auch untergeordnete Mengen weiterer copolymerisierbarer Comonomere enthalten, die in der obigen Definition als Bausteine (c) bezeichnet sind. Geeignet können hier beliebige der Co-Oligomerisation zugängliche ethylenisch ungesättigte Verbindungen sein, wie sie im einschlägigen Stand der Technik beschrieben sind, vgl. hier beispielsweise EP 418 661 A1, Spalte 3, 49 bis Spalte 4, 27. In der Regel handelt es sich hier um Comonomere, die weder eine ausgeprägte hydrophobierende Wirkung aufweisen noch hydrophilierende Gruppen im Sinne der Carboxylgruppen oder Sulfonsäuregruppen der erfindungsgemäß mitverwendeten Komponenten (b) besitzen. Werden solche Comonomere (c) in den erfindungsgemäß beschriebenen amphiphilen Co-Oligomeren mitverwendet, so beträgt ihr Anteil vorzugsweise nicht mehr als etwa 30 Gew.-% und insbesondere nicht mehr als etwa 15 Gew.-% - die Angabe Gew.-% - bezieht sich dabei auf die Summe von (a), (b) und (c).

Im Sinne der erfindungsgemäßen Lehre sind diejenigen Co-Oligomere (III) besonders bevorzugt, die frei sind von den gemäß der obigen Definition fakultativen Bausteinen (c), das heißt die ausschließlich aus den Bausteinen (a) und (b) aufgebaut sind. Diese bevorzugten Co-Oligomeren (III) enthalten dabei die Bausteine (a) und (b) in Mischungsverhältnissen von etwa 40 bis 70 Gew.-% (a) auf 60 bis 30 Gew.-% (b); die Gew.-%-Angaben beziehen sich dabei wieder die Summe von (a) und (b).

Die Co-Oligomeren (III) auf Basis der Fettcrotonate weisen bevorzugt mittlere Molgewichte (Molmassen) im Bereich von etwa 500 bis 30.000 g/Mol auf. Geeignet können dabei insbesondere Molmassen im Bereich von etwa 1.000 bis 15.000 g/Mol sein. Es hat sich gezeigt, daß innerhalb dieser vergleichsweise breiten Bereiche den unteren Werten - mittlere Molgewichte beziehungsweise Molmassen im Bereich von etwa 1.000 bis 4.000 oder 5.000 g/Mol und zweckmäßigerweise im Bereich von etwa 1.000 bis 3.000 g/Mol - besondere Bedeutung zukommen kann.

Wie angegeben sind diejenigen Co-Oligomere (III) im Rahmen der erfindungsgemäßen Lehre bevorzugt, die aus der Umsetzung von Fettcrotonaten und Maleinsäureanhydrid resultieren und deren Maleinsäureanhydrid-Bausteine durch Hydrolyse und/oder Solvolyse mit H-aktiven Komponenten in die korrespondierende Form mit freien Carboxylgruppen umgewandelt worden sind. Zur Solvolyse der Maleinsäureanhydrid-Bausteine mit H-aktiven Komponenten eignen sich insbesondere Alkohole aber auch andere Verbindungen wie Carbonsäuren, reaktiven Wasserstoff enthaltende Aminoverbindungen und dergleichen. Durch eine solche gezielte Solvolyse kann gezielt Einfluß genommen werden auf eine Verstärkung der fettenden beziehungsweise wasserabweisenden Eigenschaften der Co-Oligomeren (III). Darüber hinaus kann durch Mitverwendung hinreichend langer Kohlenwasserstoffreste das Verhältnis von oleophilen zu hydrophilen Gruppen in Richtung auf die wasserabweisenden oleophilen Elemente verschoben werden.

Der Zugang zu den Co-Oligomeren (III) ist aber auch beispielsweise auf folgende Art möglich: In einer ersten Verfahrensstufe werden die freie Crotonsäure und Maleinsäureanhydrid miteinander zur entsprechenden Oligomerverbindung umgesetzt. Anschließend wird unter Ausschluß von Wasser und raschem Austrag des Kondensationswassers eine Veresterung der Crotonsäurebestandteile mit den gewünschten Fettalkoholen beziehungsweise Fettalkoholgemischen vorgenommen. Nachfolgend wird dann die Hydrolyse beziehungsweise Solvolyse der Anhydridringe durchgeführt.

Die **Alkylendiamintetrapropionsäuren (I)** sind zur raschen und durchdringenden imprägnierenden Ausrüstung von Leder und/oder Pelzen besonders geeignet, so daß Leder zugänglich werden, die sich durch ihre Waschechtheit und auch Reinigungsechtheit (z.B. in bezug auf eine chemische Reinigung) auszeichnen. Sie sind darin ähnlichen Verbindungen, die aus dem Stand der Technik bekannt sind, überlegen. Die erfindungsgemäßen Verbindungen (I) zeichnen sich darüber hinaus durch gute Fogging-Echtheit sowie durchgute hydrophobierende Eigenschaften (Erhöhung der Wasserdichtigkeit) aus. Ein Vorteil der Verbindungen (I) besteht darin, daß sie selbst-fixierende sind, das heißt, daß im Anschluß an den Eintrag des fettenden bzw. hydrophobierenden Wirkstoffs (I) in das Leder ein anschließender zusätzlicher Fixierungsschritt nicht zwingend erforderlich ist.

Die Verbindungen (I) werden üblicherweise in wäßrigem Milieu zur fettenden Ausrüstung von Leder, insbesondere chromgegerbtem Leder, eingesetzt. Dabei kann es sich - je nach der Natur der Reste M¹ bis M⁴ beziehungsweise der - gegebenenfalls substituierten Alkylengruppe [-(CH₂)ₓ-] - um wäßrige Lösungen oder um wäßrige Dispersionen der Verbindungen (I) handeln.

Ein weiterer Gegenstand der vorliegenden Erfindung sind daher Mittel zur fettenden Ausrüstung von Ledern, Pelzen und dergleichen in Form einer wäßrigen Lösung bzw. Dispersion, die sich durch einen Gehalt an Alkylendiamintetrapropionsäuren (I) auszeichnen.

Diese **wäßrigen Angebotsformen** werden bevorzugt auf einen schwach sauren bis schwach alkalischen Bereich eingestellt. Zur Sicherstellung der Lagerstabilität kann es dabei zweckmäßig sein, Angebotsformen vorzusehen, deren wäßrige Phase durch Zugabe von anorganischen und/oder organischen Basen auf neutrale bis schwach alkalische pH-Werte eingestellt ist. Zur pH-Regulierung eignen sich an sich alle im einschlägigen Stand der Technik beschriebenen Basen. Besonders bevorzugt sind die Alkalisalze, insbesondere des Natriums und/oder Kaliums. Aber auch Ammoniumsalze oder Salze von Alkanolaminen wie Diethanolamin sind geeignete Vertreter. Bevorzugt werden die pH-Werte der wäßrigen Angebotsformen der Verbindungen (I) - oder deren Kombinationen mit den Verbindungen (II) und/oder (III) - im Bereich um pH 7 bis 8 eingestellt.

Der Wertstoffgehalt der wäßrigen Angebotsformen an den Verbindungen (I) - oder deren Kombinationen mit den Verbindungen (II) und/oder (III) - wird typischerweise im Bereich von etwa 20 bis 75 Gew.-% eingestellt. Sie lassen sich mit Wasser und/oder wäßrigen Wirkstoffabmischungen der nachfolgend geschilderten Art jederzeit aufmischen und zum praktischen Einsatz bringen.

Die Verbindungen (I) - sowie auch deren Kombinationen mit den Verbindungen (II) und/oder (III) - zeichnen sich durch selbstemulgierende Eigenschaften aus, so daß für den praktischen Einsatz der wäßrigen Angebotsformen die Anwesenheit weiterer Emulgatoren an sich entbehrlich ist.

Es kann aber gewünscht sein, daß die wäßrigen Angebotsformen der Verbindungen (I) - beziehungsweise deren Kombinationen mit den Verbindungen (II) und/oder (III) - weitere ausgewählte Emulgatoren enthalten, die beim Eintrag in insbesondere mineralgegerbte Leder und/oder Felle eine zusätzliche Fettung beziehungsweise Hydrophobierung bewirken und bevorzugt gleichzeitig über saure Gruppen im gegerbten Leder beziehungsweise Fell fixiert werden können. Ein wichtiges Beispiel für Verbindungen dieser Art sind die eingangs genannten in Wasser emulgierbaren Sulfobernsteinsäure-Halbester, die von langkettigen Fettalkoholen und/oder ihren Alkylenoxidaddukten abstammen. Ein wichtiges Beispiel für die Emulgatorenklasse der hier betroffenen Art sind C₁₈-Sulfobernsteinsäure-Halbester. Es hat sich gezeigt, daß durch die Mitverwendung solcher emulgatorartiger Hilfskomponenten - die an sich als Ausrüstungsmittel für die Lederfettung vorbekannt sind - im Sinne der erfindungsgemäßen Zielsetzung vorteilhafte Wirkungen erreicht werden können. Als Beispiele für Verbindungstypen dieser Art seien im einzelnen hier benannt:

Sulfobernsteinsäure-Halbester langkettiger Fettalkohole mit insbesondere 12 bis 24 C-Atomen und/oder deren Alkylenoxidaddukten mit bevorzugt bis zu 6 Alkylenoxidresten, entsprechende Sulfobernsteinsäure-Halbester von Fettsäuremono- und/oder -diglyceriden beziehungsweise deren Alkylenoxidaddukten mit bevorzugt bis zu 6 Alkylenoxidresten bei weiterhin bevorzugter Kettenlänge der Fettsäure(n) im Bereich C₁₂₋₂₄, langkettige Sulfofettsäuren, insbesondere entsprechende alpha-Sulfofettsäuren mit vorzugsweise 12 bis 24, insbesondere 16 bis 18 C-Atomen, wobei im Falle dieser alpha-substituierten Sulfofettsäuren die Kohlenwasserstoffreste üblicherweise gesättigt sind, sowie innenständige Sulfofettsäuren von olefinisch 1- und/oder mehrfach ungesättigten Carbonsäuren wie Ölsäure, Linolsäure, Linolensäure und dergleichen.

Die wäßrigen Angebotsformen der Verbindungen (I) - sowie auch deren Kombinationen mit den Verbindungen (II) und/oder (III) - können auch Fettungs- beziehungsweise Hydrophobierungsmittel im Sinne der Wertstoffgemische enthalten, wie sie in der eingangs zitierten EP 193 832 beschrieben sind. In dieser Ausführungsform der Erfindung werden die erfindungsgemäßen Verbindungen (I) kombiniert mit imprägnierenden und/oder hydrophobierenden Fettungsmitteln, wie Sulfobernsteinsäuremonoester-Salze mit C₁₂₋₂₄-Fettresten, in Kombination mit weiteren imprägnierenden Fettungsmitteln, ausgewählt insbesondere aus der Gruppe der oxidierten oder oxidierten und teilsulfierten C₁₈₋₂₆-Kohlenwasserstoffe oder C₃₂₋₄₀-Wachse eingesetzt. Andere Beispiele für diese zusätzlichen imprägnierenden Fettungsmittel sind Phosphorsäuremono-C₁₂₋₂₄-Alkylester, Partialester von Polycarbonsäuren wie Citronensäure-Mono-C₁₆₋₂₄-Alkylester, Partialester von Polyalkoholen wie Sorbitan-, Glycerin- oder Pentaerythrit-C₁₆₋₂₄-fettsäureester.

Eine besonders geeignete Emulgatorklasse, die im Rahmen der erfindungsgemäßen Lehre mitverwendet werden kann, sind die aus der Ausrüstung von Ledern und Pelzen mit Fettstoffen bekannten N-Acylaminosäuren, insbesondere Fettsäuresarkoside, z.B. N-Oleoyl-sarkosin, wie sie beispielsweise als Emulgatoren zum Eintrag von Silikonölen in Leder und Pelze in der EP-B-213 480 im einzelnen beschrieben sind. Geeignete Emulgatoren sind demgemäß insbesondere Salze von N-(C₉₋₂₀-Acyl)-aminosäuren, wobei entsprechenden Salzen einer Aminosäure mit 2 bis 6 C-Atomen, die mit dem Acylrest einer gesättigten oder ungesättigten Fettsäure mit 9 bis 20 C-Atomen am Aminstickstoff, der gegebenenfalls zusätzlich durch Methyl substituiert ist, besondere Bedeutung zukommt. Geeignete Salze dieser Emulgatoren sind wiederum insbesondere Alkali-, Ammonium- oder Alkanolaminsalze.

Von den N-(C₉₋₂₀-Acyl)-aminosäuren sind solche mit 2 bis 4 C-Atomen und mit der Aminogruppe in alpha-Stellung zur Carboxylgruppe besonders bevorzugt, die weiterhin am Aminstickstoffatom zusätzlich durch eine Methylgruppe substituiert sind. Davon weisen eine besonders überlegene Wirkung die Fettsäuresarkoside von gesättigten oder ungesättigten Fettsäuren mit 9 bis 20, bevorzugt 16 bis 18 C-Atomen auf. Das bevorzugte Sarkosid ist das Ölsäuresarkosid. Weiterhin sind insbesondere das N-Stearoyl-sarkosin, N-Lauroyl-sarkosin und N-Isononanoyl-sarkosin besonders geeignet und zwar jeweils in Form ihrer Alkalisalze, Ammoniumsalze oder der Salze von Mono-, Di- oder Trialkanolaminen mit insbesondere 2 bis 4 C-Atomen im Alkanolrest.

Beim Einsatz von solchen Wertstoffgemischen beträgt die Menge der erfindungsgemäß einzusetzenden Verbindungen (I) - beziehungsweise die Gesamtmenge der Verbindungen (I) bis (III) - bevorzugt wenigstens etwa 35 Gew.-% des Wertstoffgemisches und insbesondere wenigstens etwa 50 Gew.-%. Es kann jedoch zweckmäßig sein, wenigstens etwa 70 bis 80 Gew.-% des ingesamt in die auszurüstenden Leder beziehungsweise Pelzfelle einzubringenden Wertstoffgemisches auf Basis der Verbindungen (I) vorzusehen.

Der Eintrag der erfindungsgemäßen Verbindungen (I) - beziehungsweise deren Kombinationen mit den Verbindungen (II) und/oder (III) - über ihre wäßrige Angebotsform beziehungsweise ihrer Abmischungen mit den genannten weiteren Komponenten erfolgt in an sich bekannter Weise - siehe hierzu insbesondere auch die Angaben der eingangs genannten Druckschriften. Nur kurz sei daher zusammenfassend dargestellt:

Die erfindungsgemäßen Verbindungen (I) - beziehungsweise deren Kombinationen mit den Verbindungen (II) und/oder (III) - eigenen sich zur Behandlung von allen üblichen gegerbten Häuten, insbesondere entsprechendem Material, das mit Mineralgerbstoffen gegerbt worden ist. Die gegerbten Häute werden üblicherweise vor der Behandlung entsäuert. Sie können bereits vor der Behandlung gefärbt worden sein. Eine Färbung kann aber auch erst nach der erfindungsgemäß durchgeführten Behandlung vorgenommen werden.

Das zur imprägnierende Leder wird üblicherweise mit den Verbindungen (I) - beziehungsweise deren Kombinationen mit den Verbindungen (II) und/oder (III) - in wäßriger Flotte zweckmäßig bei pH-Werten von etwa 4 bis 10 und vorzugsweise bei pH 5 bis 8 und bei Temperaturen von etwa 20 bis 60°C, vorzugsweise 30 bis 50°C, während eines Zeitraumes bis zu einigen Stunden gegebenenfalls mehrstufig behandelt. Die Behandlung erfolgt beispielsweise durch Walken in einem Faß. Die Menge an der erfindungsgemäßen Verbindung (I) - beziehungsweise die Gesamtmenge der Verbindungen (I) bis (III) - in Form ihrer wäßrigen Angebotsform beträgt üblicherweise 0,1 bis 30 Gew.-%, insbesondere 1 bis 20 Gew.-% bezogen auf das Falzgewicht des Leders oder das Naßgewicht der Pelzfelle. Die Flottenlänge beträgt üblicherweise 10 bis 1.000 %, vorzugsweise 30 bis 150 %, bei Pelzfellen 50 bis 500 %.

Nach Abschluß der Behandlung mit der wäßrigen Flotte wird der pH-Wert der Behandlungsflotte durch Zusatz von Säuren in den leicht sauren Bereich verschoben. Geeignet ist insbesondere der Zusatz organischer Säuren, bevorzugt Ameisensäure. Bevorzugte pH-Werte liegen im Bereich von 3 bis 5, vorzugsweise im Bereich von etwa 3,5 bis 4.

Gewünschtenfalls kann eine Fixierung mit insbesondere Mineralgerbstoffen nachgeschaltet werden, wobei hier der Einsatz von Aluminiumsalzen, aber auch von anderen mehrwertigen Mineralsalzen, z.B. Chrom- oder Zirkonsalzen besonders bevorzugt sein kann. Wie bereits ausgeführt ist eine derartige Fixierung aber nicht zwingend erforderlich.

Ein weiterer Gegenstand der vorliegenden Erfindung ist daher ein Verfahren zur Hydrophobierung von Leder, Pelzen und dergleichen unter Verwendung von Alkylendiamintetrapropionsäuren (I), wobei man Leder bzw. Pelze in wäßriger Flotte mit Hydrophobierungsmitteln behandelt, die mindestens eine der Verbindungen (I) enthält, wobei man auf eine spätere Mineralsalzfixierung verzichtet.

### Beispiele

### 1. Herstellung von Alkylendiamintetrapropionsäuren

Alkylendiamintetraessigsäuren lassen sich nach allgemein bekannten Synthesemethoden der organischen Chemie herstellen, z. B. durch Umsetzung von Alkylendiaminen mit Acrylsäure und/oder Methacrylsäure. Die Durchführung dieses Reaktionstyps sei beispielhaft verdeutlicht anhand der Synthese von Hexamethylendiamintetrapropionsäure:

### Hexamethylendiamintetrapropionsäure

In einen Dreihalskolben mit regelbarem Innenthermometer, Tropftrichter und einem Stickstoff-Überleitungsrohr wurden 622 g Wasser sowie 194,6 g (2,70 Mol) Acrylsäure vorgelegt. Anschließend wurden langsam 108,0 g (1,35 Mol) einer 50 gew.-%igen wäßrigen Natronlauge innerhalb von 10 Minuten zugetropft. Die Temperatur der Reaktionsmischung stieg dabei auf 50 °C. Man erhitzte auf 60 °C und tropfte 75,7 g (0,67 Mol) 1,6-Diaminohexan innerhalb von 10 Minuten zu. Die dabei stattfindende Reaktion war begleitet von einer starken nebelartigen Rauchentwicklung und einem Temperaturanstieg auf 80 °C. Man erhitzte anschließend auf eine Temperatur von 90 °C und rührte die Reaktionsmischung 5 Stunden bei dieser Temperatur. Isoliert wurden 992 g einer leicht gelblich gefärbten Flüssigkeit mit folgenden Kennzahlen: Aminzahl = 70,0; Säurezahl = 74,5; Wasserwert (nach Fischer) = 68 %.

### 2. Ausführungsbeispiele

Waschechtheit, Reinigungsechtheit, Fogging-Echtheit und Wasserdichtigkeit von Ledern, die mit den Verbindungen (I) behandelt worden waren, wurden generell als gut eingestuft.

Die Wasserdichtigkeit von mit Alkylendiamintetrapropionsäuren behandelten Ledern wurde dabei - als Inidkator für die hydrophobierenden Eigenschaften der erfindungsgemäß einzusetzenden Verbindungen (I) - mit einem Bally-Penetrometer gemäß Meßmethode IUP 10 der Internationalen Union der Leder-Chemiker-Verbände (Kommission für physikalische Lederprüfung) geprüft. Die Methode ist publiziert in: "Das Leder", 1961, 12. Jahrgang, Seiten 36-40.

Als Kenngröße für die Güte der Hydrophobierung diente dabei die Wasserdurchtrittszeit. Die Stauchung der Leder betrug bei diesen Untersuchungen 15 %.

Es stellte sich heraus, daß bei Verbindungen wie Tetrabutylendiamintetrapropionsäure und Hexamethylendiamintetrapropionsäure die Wasserdurchtrittszeiten sehr gering waren. Derartige Verbindungen eignen sich in der Praxis nicht zur Hydrophobierung. Die erfindungsgemäß einzusetzenden Verbindungen (I) wiesen dagegen befriedigende bis sehr gute Werte in Bezug auf die Wasserdurchtrittszeiten auf (z. B. > 6 Stunden für (HO₂C-CH₂-CH₂)₂N-(CH₂)ₓ-(CH₂-CH₂-CO₂H)₂ mit x = 12 bzw. 16). Diese Ergebnisse sind bemerkenswert, zumal die Stauchung der Leder bei den Penetrometertests auf 15 % eingestellt wurde, was bedeutet, daß die Lederfasern unter diesen Prüfbedingungen sehr hohen Stauchungskräften ausgesetzt sind.

## Patentansprüche

1. Verwendung von Alkylendiamintetrapropionsäuren der allgemeinen Formel (I) 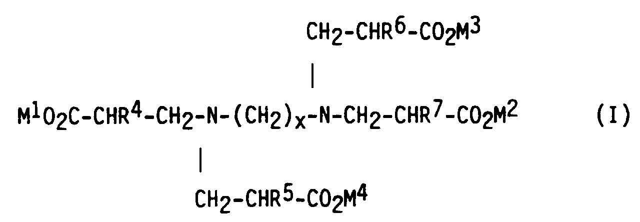 worin
- die Reste R⁴ bis R⁷ unabhängig voneinander Wasserstoff oder eine Methylgruppe und
- die Reste M¹ bis M⁴ unabhängig voneinander Wasserstoff, Alkalimetall, Erdalkalimetall, Ammonium, Alkylammonium oder Alkanolammonium und
- x eine Zahl von 12 bis 36 bedeuten und
- wobei die Alkylengruppe -(CH₂)ₓ- gegebenenfalls durch ein oder mehrere Alkyl-, Aryl- oder Alkarylgruppen substituiert sein kann, mit der Maßgabe, daß die Gesamtzahl der C-Atome dieser Substituenten 1 bis 40 beträgt,
zur fettenden Ausrüstung von Leder.

2. Verwendung nach Anspruch 1, wobei die Reste R¹ bis R⁴ - unabhängig voneinander - Wasserstoff oder ein Alkalimetall bedeuten.

3. Verwendung nach Anspruch 1 oder 2, wobei man die Alkylendiamintetrapropionsäure (I) in Kombination mit
i) mindestens einem Aminopropionsäurederivat der allgemeinen Formel (II) 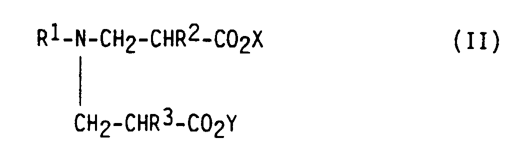 worin
- der Rest R¹ eine gesättigte, geradkettige oder verzweigte Alkylgruppe mit 8 bis 22 C-Atomen,
- die Reste R² und R³ unabhängig voneinander Wasserstoff oder eine Methylgruppe und
- die Reste X und Y unabhängig voneinander Wasserstoff, Alkalimetall, Erdalkalimetall, Ammonium, Alkylammonium oder Alkanolammonium bedeuten
und /oder
ii) mindestens einem wasserdispergierbaren und/oder wasseremulgierbaren Co-Oligomeren (III) aus
a) Fettcrotonaten und
b) radikalisch copolymerisierbaren hydrophilen ethylenisch ungesättigen Säuren und/oder deren Anhydriden, die auch
c) untergeordnete Mengen weiterer copolymerisierbarer Comonomere enthalten können,
einsetzt.

4. Verwendung nach einem der Ansprüche 1 bis 3, wobei man die Verbindungen (I) - beziehungsweise deren Kombination mit mindestens einer der Verbindungen (II) und/oder (III) - in wäßriger Angebotsform, d.h. in Form einer wäßrigen Lösung bzw. Dispersion, einsetzt.

5. Verwendung nach Anspruch 4, wobei man den pH-Wert der wäßrigen Angebotsform im Bereich von schwach sauer bis schwach alkalisch einstellt.

6. Verfahren zur Hydrophobierung von Leder, Pelzen und dergleichen unter Verwendung von Alkylendiamintetrapropionsäuren (I), wobei man Leder bzw. Pelze in wäßriger Flotte mit Hydrophobierungsmitteln behandelt, die mindestens eine der Verbindungen (I) gemäß Anspruch 1 enthält, dadurch gekennzeichnet, daß man auf eine spätere Mineralsalzfixierung verzichtet.

7. Mittel zur fettenden Ausrüstung von Ledern, Pelzen und dergleichen in Form einer wäßrigen Lösung oder Dispersion, dadurch gekennzeichnet, daß sie Alkylendiamintetrapropionsäuren (I) nach einem der Ansprüche 1 bis 3 enthalten.

## Claims

1. The use of alkylenediaminotetrapropionic acids corresponding to general formula (I): 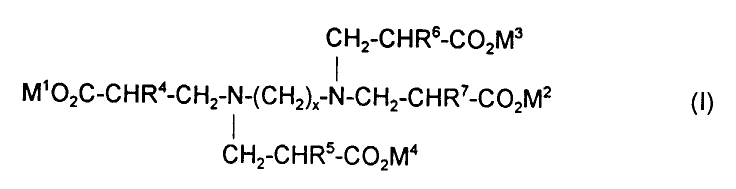 in which
- R⁴ to R⁷ independently of one another represent hydrogen or a methyl group,
- M¹ to M⁴ independently of one another represent hydrogen, alkali metal, alkaline earth metal, ammonium, alkylammonium or alkanolammonium and
- x is a number of 12 to 36,
- the alkylene group -(CH₂)ₓ- optionally being substituted by one or more alkyl, aryl or alkaryl groups, with the proviso that the total number of carbon atoms in these substituents is between 1 and 40,
for the oiling of leather.

2. The use claimed in claim 1, characterized in that the substituents R¹ to R⁴ independently of one another represent hydrogen or an alkali metal.

3. The use claimed in claim 1 or 2, characterized in that the alkylenediaminotetrapropionic acid (I) is used in combination with
i) at least one aminopropionic acid derivative corresponding to general formula (II): 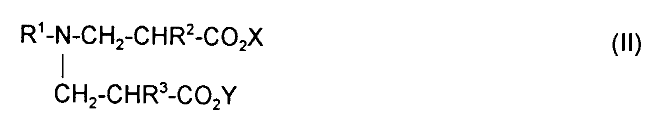 in which
- R¹ is a saturated, linear or branched alkyl group containing 8 to 22 carbon atoms,
- R² and R³ independently of one another represent hydrogen or a methyl group and
- X and Y independently of one another represent hydrogen, alkali metal, alkaline earth metal, ammonium, alkylammonium or alkanolammonium
and/or
ii) at least one water-dispersible and/or water-emulsifiable co-oligomer (III) of
a) fatty crotonates and
b) radical-copolymerizable hydrophilic, ethylenically unsaturated acids and/or anhydrides thereof which may also contain
c) small quantities of other copolymerizable comonomers.

4. The use claimed in any of claims 1 to 3, characterized in that the compounds (I) - or combinations thereof with at least one of the compounds (II) and/or (III) - are used in the form of an aqueous formulation, i.e. in the form of an aqueous solution or dispersion.

5. The use claimed in claim 4, characterized in that the aqueous formulation is adjusted to a mildly acidic to mildly alkaline pH value.

6. A process for hydrophobicizing leather, skins and the like using alkylenediaminotetrapropionic acids (I), in which leather or skins are treated in aqueous medium with hydrophobicizing agents containing at least one of the compounds (I) according to claim 1, characterized in that the hydrophobicizing agents are not subsequently fixed with mineral salts.

7. Formulations for the oiling of leathers, skins and the like in the form of an aqueous solution or dispersion, characterized in that they contain alkylenediaminotetrapropionic acids (I) according to any of claims 1 to 3.

## Revendications

1. Utilisation d'acides alkylènediaminotétrapropioniques de la formule générale (I) 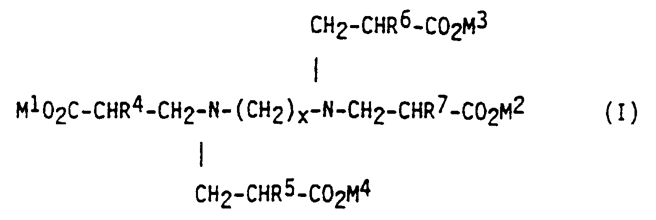 dans laquelle
- les radicaux R⁴ à R⁷ représentent indépendamment les uns des autres l'hydrogène ou un groupe méthyle et
- les radicaux M¹ à M⁴ correspondent indépendamment les uns des autres à l'hydrogène, à un métal alcalin, à un métal alcalino-terreux, à un ammonium, à un alkylammonium ou à un alcanolammonium et
- x est un nombre de 12 à 36 et
- dans laquelle le groupe alkylène -(CH₂)ₓ- peut être substitué le cas échéant par un ou par plusieurs groupes alkyle, aryle ou alkaryle, à condition que le nombre total des atomes de C de ces substituants atteigne 1 à 40,
pour mettre des cuirs en huile

2. Utilisation selon la revendication 1, dans laquelle les radicaux R¹ à R⁴ représentent indépendamment les uns des autres l'hydrogène ou un métal alcalin.

3. Utilisation selon la revendication 1 ou 2, dans laquelle on met en oeuvre l'acide alkylènediaminotétrapropionique (I) en association avec
i) au moins un dérivé d'acide aminopropionique de la formule générale (II) 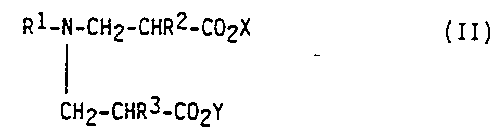 dans laquelle
- le radical R¹ représente un groupe alkyle saturé, à chaîne droite ou ramifiée comportant 8 à 22 atomes de C,
- les radicaux R² et R³ correspondent indépendamment l'un de l'autre à l'hydrogène ou à un groupe méthyle et
- les radicaux X et Y représentent indépendamment l'un de l'autre l'hydrogène, un métal alcalin, un métal alcalino-terreux, un ammonium, un alkylammonium ou un alcanolammonium
et/ou avec
ii) au moins un co-oligomère dispersible et/ou émulsionnable dans l'eau (III) à base de
a) des crotonates gras et
b) des acides hydrophiles, copolymérisables par voie radicalaire, à insaturation éthylénique et/ou des anhydrides de ceux-ci, qui peuvent également renfermer
c) des quantités secondaires d'autres comonomères copolymérisables.

4. Utilisation selon une des revendications 1 à 3, dans laquelle on met en oeuvre les composés (I) - ou leur association avec au moins un des composés (II) et/ou (III) - sous une présentation aqueuse, c'est-à-dire sous la forme d'une solution ou d'une dispersion aqueuse.

5. Utilisation selon la revendication 4, dans laquelle on ajuste le pH de la présentation aqueuse dans la plage légèrement acide à légèrement alcaline.

6. Procédé pour l'imperméabilisation du cuir, des peaux et des matières similaires, en utilisant des acides alkylène-diaminotétrapropioniques (I), dans lequel on traite les cuirs ou les peaux dans un bain aqueux par des agents imperméabilisants, qui renferment au moins un des composés (I) selon la revendication 1, caractérisé en ce que l'on renonce à une fixation ultérieure par des sels minéraux.

7. Agents pour mettre en huile des cuirs, des peaux et des matières similaires, sous la forme d'une solution ou d'une dispersion aqueuse, caractérisés en ce qu'ils renferment des acides alkylènediaminotétrapropioniques (I), selon une des revendications 1 à 3.
